Europäisches Patentamt

European Patent Office

Office européen des brevets

(11) Veröffentlichungsnummer: **0 457 074 A1**

(12) **EUROPÄISCHE PATENTANMELDUNG**

(21) Anmeldenummer: 91106659.5

(22) Anmeldetag: 25.04.91

(51) Int. Cl.5: **A61M 1/10**

(30) Priorität: 18.05.90 DE 4016013

(43) Veröffentlichungstag der Anmeldung:
21.11.91 Patentblatt 91/47

(84) Benannte Vertragsstaaten:
AT BE CH DE DK ES FR GB GR IT LI LU NL SE

(71) Anmelder: Forschungsgesellschaft für
Biomedizinische Technik e.V.
Pauwelsstrasse 30
W-5100 Aachen(DE)

(72) Erfinder: Knierbein, Bernd, Dr.-Ing.
Auf der Komm 8
W-5102 Würselen(DE)
Erfinder: Reul, Helmut, Prof. Dr.-Ing.
Akazienstrasse 65
W-5160 Düren(DE)
Erfinder: Rau, Günter, Univ.-Prof. Dr. rer. nat.
Fuchserde 50
W-5100 Aachen(DE)

(74) Vertreter: Döring, Wolfgang, Dr. Ing.
Mörikestrasse 18
W-4000 Düsseldorf 30(DE)

(54) **Verdrängerpumpe zur Förderung von Blut.**

(57) Es werden eine Verdrängerpumpe zur Förderung von Blut und ein Verfahren zu deren Herstellung beschrieben. Die Pumpe besitzt ein Pumpengehäuse (1), in das die Gehäuse der Einlaß- und Auslaßklappe (4,5) nahtlos integriert sind. Hierdurch werden Naht- bzw. Stoßstellen mit ihren nachteiligen Auswirkungen auf das Blut vermieden. Bei dem Herstellverfahren für die Verdrängerpumpe (1) wird das Pumpengehäuse zusammen mit den beiden Klappengehäusen in einem einzigen Formvorgang hergestellt. In weiteren Verfahrensschritten werden die Klappenschließkörper und die blutseitige Membran nahtlos angeformt.

FIG. 2

Die vorliegende Erfindung betrifft eine Verdrängerpumpe zur Förderung von Blut mit einem Pumpengehäuse, einer als Förderorgan dienenden, im Pumpengehäuse angeordneten Membran, die das Pumpengehäuse in eine Blutkammer und eine Kammer für das Fördermedium unterteilt, einem Einlaßstutzen am Pumpengehäuse und einem Auslaßstutzen am Pumpengehäuse.

Derartig ausgebildete Blutpumpen sind bekannt. Sie arbeiten mit einem gasförmigen oder flüssigen Antriebsmedium, das durch die flexible Membran vom Blut getrennt ist. Diese Membran ist schlauchförmig, flach, halbkugelförmig oder sackförmig ausgebildet und so in dem festen Pumpengehäuse untergebracht, daß das Blut von dem antriebsseitig ein- bzw. ausströmenden Antriebsmedium verdrängt bzw. angesaugt wird.

Derartige Blutpumpen werden mit separat ausgebildeten Ein- und Auslaßklappen betrieben, für die üblicherweise Herzklappenprothesen eingesetzt werden. Die Klappen werden dabei am Einlaß- und Auslaßstutzen des Pumpengehäuses befestigt.

Für alle Verdränger-Blutpumpen sind die Ein- und Auslaßklappen von entscheidender strömungsmechanischer bzw. hämodynamischer Bedeutung. Aus der Befestigung der Klappen an den Stutzen des Pumpengehäuses resultieren jedoch zwangsläufig Übergangs- bzw. Stoßstellen, die entsprechende strömungsmechanische bzw. hämodynamische Nachteile mit sich bringen. So resultieren thrombotische Ablagerungen, so daß beim Einsatz von derartigen Pumpen permanent gerinnungshemmende Mittel eingesetzt werden müssen. Es liegen somit keine übergangsfreien und stoßarmen Oberflächen vor, was zu entsprechenden Blutschädigungen führt.

Der Erfindung liegt die Aufgabe zugrunde, eine Verdrängerpumpe zur Förderung von Blut der angegebene Art zu schaffen, die auf der Blutseite besonders strömungsgünstig ausgebildet ist.

Diese Aufgabe wird erfindungsgemäß bei einer Verdrängerpumpe der gattungsgemäßen Art dadurch gelöst, daß der Einlaßstutzen mit dem Gehäuse einer Einlaßklappe und der Auslaßstutzen mit dem Gehäuse einer Auslaßklappe einstückig ausgebildet sind.

Bei der erfindungsgemäßen Lösung sind somit beide Klappengehäuse unmittelbar in das Pumpengehäuse integriert, so daß sich zwischen Ein- und Auslaßstutzen einerseits und den Klappengehäusen andererseits keine Stoß- und Übergangsstellen mehr ergeben. Es liegen daher im Übergangsbereich zwischen Klappe und Stutzen ablösungsfreie Störungsverhältnisse vor, die zu keinen Blutschädigungen, insbesondere thrombotischen Ablagerungen, führen. Die beiden Klappengehäuse bilden daher Verlängerungen des Einlaß- und Auslaßstutzens.

Bei einer speziellen Ausführungsform der erfindungsgemäßen Pumpe sind die Einlaß- und Auslaßklappe als Bulbenklappen ausgebildet, wobei die jeweiligen Bulben einstückig mit dem Einlaß- und Auslaßstutzen ausgebildet sind. Hierbei bilden die Klappengehäuse bulbenförmige Ausbauchungen der jeweiligen Stutzen. Auch hier sind glatte Übergangsflächen zwischen Klappengehäuse und Stutzen vorgesehen, was die vorstehend geschilderten Vorteile mit sich bringt.

Bei den heute im Handel erhältlichen Blutpumpen werden nahezu ausschließlich mechanische Kippscheibenklappen verwendet, die nicht für diesen speziellen Anwendungsfall entwickelt wurden. Durch die unphysiologische Strömungsführung im Klappenbereich wird das Blut infolge überhöhter Schubspannungen sowohl hämolytisch geschädigt als auch präthrombotisch gereizt. Der in solchen Fällen typische Weg des Blutes führt aus Bereichen hoher Schubspannungen in Rezirkulationsgebiete (Totwassergebiete). Diese fluiddynamisch zeitweise abgeschlossenen Räume ermöglichen analog einem Reagenzgefäß die Durchmischung ohne Verdünnung von geschädigten und intakten Plättchen und bilden so die Grundlage zum thrombotischen Entstehungsprozeß. In ungestörter laminarer Strömung würde diese verhältnismäßig kleine Blutschädigung infolge rascher Verdünnung der aktivierenden Mediatoren ohne Folgen bleiben. Mit anderen Worten, auch durch die Art der bisher verwendeten Klappen, insbesondere die Ausbildung des Schließkörpers, wird das Blut geschädigt. Ein weiterer Nachteil von derartigen mechanischen Klappen sind das harte Schließen und die damit verbundenen Quer- und Längsbeschleunigungen der gesamten Pumpe sowie eine entsprechende Geräuschentwicklung.

Zur Behebung dieser Nachteile wird in Weiterbildung der Erfindung vorgeschlagen, daß der Schließkörper der Klappe an das zugehörige Klappengehäuse angeformt ist. Hierdurch wird auch zwischen Klappengehäuse und Schließkörper ein glatter Übergang erreicht, so daß die vorstehend aufgezeigten Nachteile, insbesondere das Entstehen von Totwassergebieten, vermieden werden. Bei dieser Ausführungsform sind somit Pumpengehäuse, Klappengehäuse und Schließkörper der Klappe einstückig ausgebildet.

Die Klappe weist als Schließkörper vorzugsweise mindestens ein Klappensegel auf. Bei einer besonders bevorzugten Ausführungsform sind drei Klappensegel vorhanden, die jeweils an die Innenseite des Klappengehäuses angeformt sind. Eine solche Klappe zeichnet sich durch besonders gute Strömungscharakteristika aus. Bei einer Bulbenklappe sind dabei die Segel vorzugsweise an der Übergangsstelle zwischen dem Bulbus und dem zylindrischen Gehäusestutzen angeformt.

Zur Vereinfachung der Herstellung ist das Pumpengehäuse zweckmäßigerweise zweigeteilt, wobei beide Gehäusehälften über eine Spanneinrichtung miteinander verbunden sind. Dabei weist vorzugsweise die obere Gehäusehälfte die mit den integrierten Klappen versehenen Stutzen für die Blutzuführung und Blutabführung auf, während die untere Gehäusehälfte vorzugsweise einen Stutzen zur Zu- und Abführung des Fördermediums (Luft) besitzt. Als Spanneinrichtung findet vorzugsweise eine auf einen Gewindering aufgeschraubte Spannmutter Verwendung, die zwei Ringflansche der Gehäusehälften gegeneinander preßt. Durch diesen Preßvorgang wird zweckmäßigerweise die Membran mit eingepreßt, indem deren radial äußere Bereiche mit Feder-Nut-Verbindungen zwischen die beiden Gehäuseflansche geführt sind.

Die das Pumpengehäuse unterteilende Membran besitzt zweckmäßigerweise eine blutseitige Lage und eine dem Fördermedium zugewandte Lage. Beide Lagen sind vorzugsweise voneinander getrennt und liegen nur lose aneinander an, wobei zwischen beiden eine Gleitschicht, beispielsweise aus Graphit, angeordnet ist. Dabei kann die dem Fördermedium zugewandte Lage aus dem gleichen Material wie das Pumpengehäuse bestehen, während die blutseitige Lage aus einem speziellen biokompatiblen Material besteht.

Wie bereits vorstehend erläutert, wird die Membran vorzugsweise dadurch gehalten, daß sie zwischen die beiden Gehäusehälften eingespannt ist. Dies betrifft in Weiterbildung der Erfindung nur die dem Fördermedium zugewandte Lage der Membran, während die blutseitige Membranlage an die Innenseite des Pumpengehäuses angeformt ist. Bei dieser Ausführungsform ist daher auch die blutseitige Membranlage einstückig mit dem Pumpengehäuse ausgebildet. Pumpengehäuse, Stutzen, Klappengehäuse, Klappenschließkörper und blutseitige Membran bilden dabei hierbei eine Einheit.

Vorzugsweise sind Pumpengehäuse, Stutzen und Klappengehäuse auf ihren mit Blut in Berührung tretenden Flächen mit einem biokompatiblen Material beschichtet. Wenn auch die Klappenschließkörper (Segel) aus einem biokompatiblen Material bestehen, sind somit sämtliche mit Blut in Berührung tretenden Flächen der Verdrängungspumpe einschließlich der Klappen mit biokompatiblem Material versehen, so daß sich das Material der Pumpe nicht schädigend auf das Blut auswirkt.

Zweckmäßigerweise bestehen die relativ festen Bestandteile der Pumpe, nämlich das Pumpengehäuse, die Stutzen und die Klappengehäuse, aus thermoplastischem Polyurethan, das sich für diesen Einsatzzweck als besonders geeignet erwiesen hat. Als biokompatibles Material für die Herstellung der blutseitigen Membranlage und der Klappenschließkörper sowie für die Beschichtung der übrigen mit Blut in Kontakt tretenden Flächen kommt vorzugsweise in einem geeigneten Lösungsmittel lösbares biokompatibles Polyurethan zum Einsatz.

Insgesamt wird somit erfindungsgemäß eine Verdrängerpumpe mit nahtlos integrierten Ein- und Auslaßklappengehäusen sowie insbesondere mit nahtlos integrierter blutseitiger Membranlage und nahtlos integrierten Klappenschließkörpern vorgeschlagen. Die Blutpumpe ist auf der Blutseite besonders strömungsgünstig ausgebildet und weist insbesondere weichschließende Klappen auf. Alle blutführenden Bauteile können mit ein und demselben biokompatiblen flexiblen Material beschichtet oder aus diesem hergestellt sein.

Die Erfindung betrifft ferner ein Verfahren zur Herstellung einer Verdrängerpumpe der erfindungsgemäßen Art.

Wie erwähnt, ist es bekannt, für derartige Verdrängerpumpen separat hergestellte Klappen zu verwenden und diese Klappen mit den beiden Stutzen des Pumpengehäuses zu verbinden, beispielsweise zu verklemmen oder zu verkleben. Das erfindungsgemäße Verfahren wendet sich von dieser bekannten Lehre ab und schlägt in seiner allgemeinsten Ausführungsform die Herstellung des Pumpengehäuses mit den Klappengehäusen in einem einzigen Formvorgang vor.

Das Gehäuse wird dabei vorzugsweise über eine Positivform, die im Stutzenbereich die Gehäuse für die Aufnahme der Klappenschließorgane besitzt, in einem Arbeitsgang geformt, vorzugsweise thermogeformt.

In Weiterbildung umfaßt das erfindungsgemäße Verfahren des weiteren den Schritt des Anformens der Klappenschließorgane durch Antauchen. Hierbei wird zweckmäßigerweise gleichzeitig mit dem Anformen des Einlaßklappenschließorganes die Innenseite des Pumpengehäuses mit dem Material für die Klappenschließorgane beschichtet. Wie vorstehend erwähnt, handelt es sich hierbei um ein biokompatibles Material.

Ähnlich wie bei dem Einlaßklappenschließorgan wird auch das Auslaßklappenschließorgan durch Antauchen an das entsprechende Klappengehäuse angeformt.

Die blutseitige Membranlage wird vorzugsweise durch Anformen an das Pumpengehäuse hergestellt.

Bei einer besonders bevorzugten Ausführungsform des erfindungsgemäßen Verfahrens wird in der folgenden Reihenfolge vorgegangen: Zuerst wird das Pumpengehäuse mit integrierten Klappengehäusen geformt, wonach das Einlaßklappenschließorgan durch Antauchen hergestellt und an das Einlaßklappengehäuse angeformt wird. Hierbei erfolgt gleichzeitig eine Beschichtung der Innenseite des Pumpengehäuses. Im nächsten Verfahrensschritt wird das Auslaßklappenschließorgan durch Antauchen hergestellt und an das Auslaßklappengehäuse angeformt, wonach an die mit den fertigen Klappen versehene Pumpengehäusehälfte im nächsten Fertigungsschritt die blutseitige Membran angetaucht wird. Die Klappen müssen hierfür abgedeckt werden, um eine Beschädigung durch die Lösungsmittelatmosphäre beim Antauchvorgang zu unterbinden. Die Membran selbst wird durch mehrfaches Tauchen einer Kugelkalotte vorgefertigt. Beim letzten Tauchvorgang wird die Membran im feuchten Zustand mit der anderen Gehäusehälfte gefügt. Dies geschieht in Verbindung mit dem Einspannen der luftseitigen Membran und dem Festspannen der beiden Gehäusehälften gegeneinander.

Ähnlich wie bei dem Einlaßklappenschließorgan wird auch das Auslaßklappenschließorgan durch Antauchen an das entsprechende Klappengehäuse angeformt.

Die blutseitige Membranlage wird vorzugsweise durch Anformen an das Pumpengehäuse hergestellt.

Bei einer besonders bevorzugten Ausführungsform des erfindungsgemäßen Verfahrens wird in der folgenden Reihenfolge vorgegangen: Zuerst wird das Pumpengehäuse mit integrierten Klappengehäusen geformt, wonach das Einlaßklappenschließorgan durch Antauchen hergestellt und an das Einlaßklappengehäuse angeformt wird. Hierbei erfolgt gleichzeitig eine Beschichtung der Innenseite des Pumpengehäuses. Im nächsten Verfahrensschritt wird das Auslaßklappenschließorgan durch Antauchen hergestellt und an das Auslaßklappengehäuse angeformt, wonach die blutseitige Membranlage durch Antauchen hergestellt und an das Pumpengehäuse angeformt wird.

Das Pumpengehäuse wird zweckmäßigerweise in zwei Hälften hergestellt, wobei die beiden Hälften nach der Anbringung der Klappenschließorgane und der Membran miteinander verspannt werden. Wenn die Membran aus einer blutseitigen Lage und einer dem Fördermedium zugewandten Lage besteht, wird hierbei zweckmäßigerweise so vorgegangen, daß die dem Fördermedium zugewandte Membranlage zuerst einseitig eingespannt wird, während die blutseitige Membranlage ebenfalls zuerst einseitig angeformt wird. Es erfolgt dann ein Zusammenfügen der beiden Gehäusehälften, wobei die andere Seite der dem Fördermedium zugewandten Membranlage eingespannt und die andere Seite der blutseitigen Membranlage im noch feuchten Zustand mit dem Gehäuse gefügt wird.

Die Erfindung wird nachfolgend anhand eines Ausführungsbeispiels in Verbindung mit der Zeichnung im einzelnen erläutert. Es zeigen:

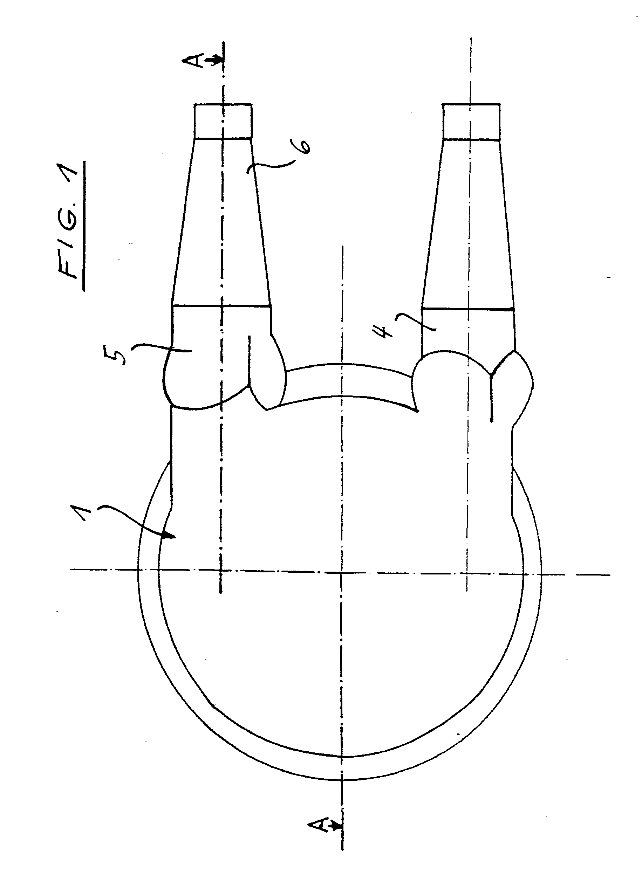

Figur 1   eine Draufsicht auf eine Ausführungsform einer Verdrängerpumpe mit integrierten Klappen; und

Figur 2   einen Schnitt durch die Verdrängerpumpe der Figur 1 entlang Linie A-A in Figur 1.

Die in den Figuren dargestellte Verdrängerpumpe zur Förderung von Blut besteht aus einem Pumpengehäuse 1, das sich aus einer oberen und einer unteren Gehäusehälfte 2, 3 zusammensetzt. Das Pumpengehäuse ist mit einer nahtlos integrierten Einlaß- und Auslaßklappe 4, 5 versehen. An diese Klappen sind entsprechende Reduzierstücke 6 angeschlossen, die mit entsprechenden Leitungen in Verbindung gebracht werden können.

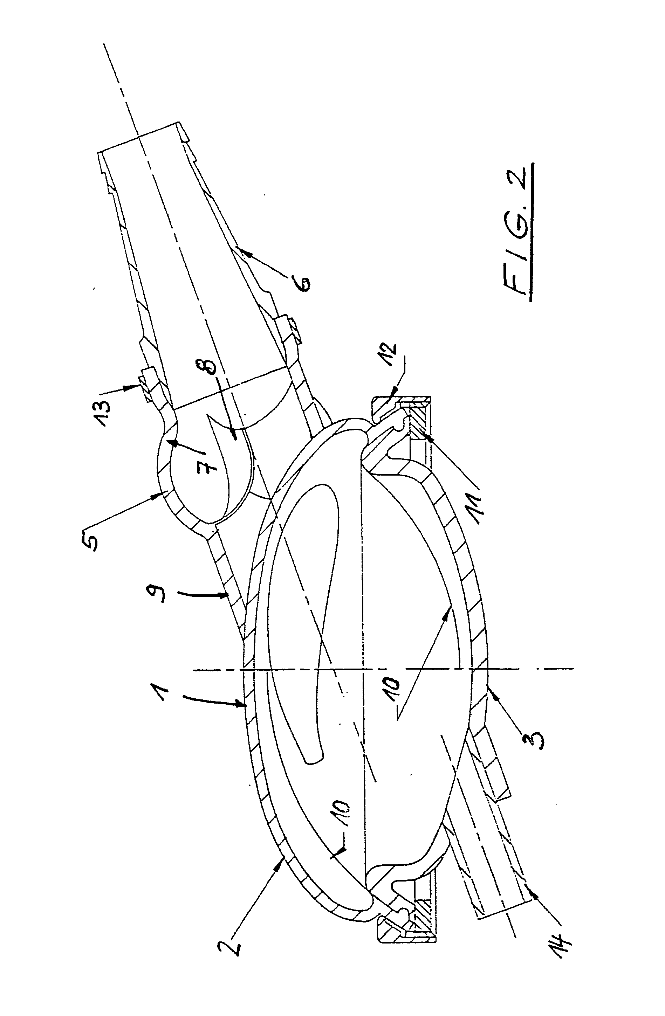

Figur 2 zeigt den Aufbau des Pumpengehäuses 1 im einzelnen. Die beiden Hälften 2, 3 des Pumpengehäuses, die jeweils etwa halbkugelförmig ausgebildet sind, sind mit Endflanschen versehen, die aneinander liegen. Der Endflansch der oberen Hälfte 2 weist dabei eine Feder auf, die in eine entsprechende Nut des Endflansches der unteren Hälfte 3 eingreift. Diese beiden Endflansche sind mit Hilfe eines Gewinderinges 11 und einer Spannmutter 12 gegeneinander verspannt, so daß eine dichte Gehäuseeinheit gebildet wird. Mit zwischen die Flansche eingespannt ist eine Membran 10, die die Pumpenkammer im Gehäuse 1 in zwei Hälften unterteilt. Die Membran ist in der Figur links in ihrem endsystolischen und in der Figur rechts in ihrem enddiastolischen Zustand dargestellt.

Die Membran 10 unterteilt die Pumpenkammer in eine Luftkammer und eine Blutkammer. In die Luftkammer mündet ein Luftstutzen 14, mittels dem wechselweise Druck- und Unterdruck auf die Luftseite der Membran geführt werden. Auf diese Weise erfolgt eine Förderung des durch die Blutkammer strömenden Blutes. Die Blutkammer steht dabei über einen Einlaßstutzen und einen Auslaßstutzen 9 und die entsprechende Einlaß- und Auslaßklappe 4, 5 mit einem blutführenden Leitungssystem in Verbindung.

Wie man Figur 2 entnehmen kann, ist das Gehäuse 7 der Auslaßklappe 5 zusammen mit dem Auslaßstutzen und der oberen Hälfte 2 des Pumpengehäuses 1 einstückig ausgebildet, so daß keine Naht- bzw. Stoßstelle dazwischen vorhanden ist. Gleiches trifft auf das Gehäuse der Einlaßklappe zu, das einstückig mit dem Einlaßstutzen und der oberen Hälfte 2 des Pumpengehäuses ausgebildet ist. Bei der hier dargestellten Ausführungsform sind Einlaß- und Auslaßklappe als Bulbenklappe ausgebildet, so daß die entsprechenden Klappengehäuse gegenüber den zugehörigen Stutzen erweitert sind. Als Schließkörper der Klappen finden drei Segel 8 Verwendung. Auch die Schließkörper sind nahtlos in das jeweilige Klappengehäuse integriert. Dies trifft auch auf die blutseitige Lage der Membran 10 zu, die an die Innenseite des Pumpengehäuses angeformt ist.

Die beiden Reduzierstücke 6 sind über einen geeigneten Schlauchbinder 13 mit dem zugehörigen Klappengehäuse verbunden.

Zur Herstellung der Pumpe mit integrierten Klappen wird wie folgt vorgegangen:

Zuerst wird die obere Gehäusehälfte 2 zusammen mit den beiden Klappengehäusen in einem Arbeitsgang thermogeformt. Hierzu wird eine mehrfach zerlegbare Positivform verwendet, die im Stutzenbereich die Bulben für die Aufnahme der Klappensegel besitzt.

In entsprechender Weise wird die untere Gehäusehälfte 3 geformt.

Die fertiggeformte Gehäusehälfte 2 wird dann von Materialresten freigeschnitten, auf einen Ring gespannt und fixiert. An diesem Ring wird das Tauchwerkzeug für die Segel der Einlaßklappe befestigt. Durch Ausschwenken mit PUR-Lösung werden sowohl die Klappensegel hergestellt als auch das Pumpengehäuse von innen beschichtet. Der Abfluß der überschüssigen Kunststofflösung erfolgt dabei durch die Auslaßöffnung, die z. T. abgedeckt wird, um dort die Bulbenkontur durch die mehrfachen Beschichtungen nicht zu verändern.

Nach Austrocknung werden die Segelkanten der Einlaßklappe aufgeschnitten oder aufgeschmolzen, um dann das Klappenwerkzeug zu entfernen. Dies kann auch durch Laserschneiden geschehen.

Hiernach erfolgt die Fertigung der Auslaßklappensegel. Ähnlich wie bei der Einlaßklappe kann nun das Klappenwerkzeug am Ring im Auslaßbulbus befestigt werden. Durch Ausschwenken des Bulbus und anschließendes Taumeln können nun ähnlich wie bei der bisherigen Klappe die Segel hergestellt werden. Nach der Trocknung werden die Segelkanten aufgeschnitten bzw. aufgeschmolzen, und das Klappenwerkzeug wird entfernt.

An die mit den fertigen Klappen versehenen Pumpengehäusehälfte wird im nächsten Fertigungsschritt die blutseitige Membran angetaucht. Die Klappen müssen hierfür abgedeckt werden, um eine Beschädigung durch die Lösungsmittelatmosphäre beim Antauchvorgang zu unterbinden. Die Membran selbst wird durch mehrfaches Tauchen einer Kugelkalotte vorgefertigt. Beim letzten Tauchvorgang wird die Membran im feuchten Zustand mit der anderen Gehäusehälfte gefügt. Dies geschieht in Verbindung mit dem Einspannen der luftseitigen Membran und dem Festspannen der beiden Gehäusehälften gegeneinander.

## Patentansprüche

1. Verdrängerpumpe zur Förderung von Blut mit einem Pumpengehäuse, einer als Förderorgan dienenden, im Pumpengehäuse angeordneten Membran, die das Pumpengehäuse in eine Blutkammer und eine Kammer für das Fördermedium unterteilt, einem Einlaßstutzen am Pumpengehäuse und einem Auslaßstutzen am Pumpengehäuse, dadurch gekennzeichnet, daß der Einlaßstutzen mit dem Gehäuse einer Einlaßklappe (4) und der Auslaßstutzen (9) mit dem Gehäuse (7) einer Auslaßklappe (5) einstückig ausgebildet sind.

2. Verdrängerpumpe nach Anspruch 1, dadurch gekennzeichnet, daß Einlaß- und Auslaßklappe (4, 5) als Bulbenklappen ausgebildet sind, wobei die jeweiligen Bulben einstückig mit dem Einlaß- und Auslaßstutzen (9) ausgebildet sind.

3. Verdrängerpumpe nach Anspruch 1 oder 2, dadurch gekennzeichnet, daß der Schließkörper (8) der jeweiligen Klappe (4, 5) an das zugehörige Klappengehäuse (7) angeformt ist.

4. Verdrängerpumpe nach Anspruch 3, dadurch gekennzeichnet, daß die Klappe (5) als Schließkörper (8) mindestens ein Klappensegel aufweist.

5. Verdrängerpumpe nach einem der vorangehenden Ansprüche, dadurch gekennzeichnet, daß das Pumpengehäuse (1) zweigeteilt ist, wobei beide Gehäusehälften (2, 3) über eine Spanneinrichtung (11, 12) miteinander verbunden sind.

6. Verdrängerpumpe nach einem der vorangehenden Ansprüche, dadurch gekennzeichnet, daß die Membran (10) eine blutseitige Lage und eine dem Fördermedium zugewandte Lage aufweist.

7. Verdrängerpumpe nach Anspruch 6, dadurch gekennzeichnet, daß die blutseitige Membranlage an die Innenseite des Pumpengehäuses (1) angeformt ist.

8. Verdrängerpumpe nach einem der vorangehenden Ansprüche, dadurch gekennzeichnet, daß Pumpengehäuse (1), Stutzen (9) und Klappengehäuse (7) auf ihren mit Blut in Berührung tretenden Flächen mit einem biokompatiblen Material beschichtet sind.

9. Verdrängerpumpe nach einem der vorangehenden Ansprüche, dadurch gekennzeichnet, daß die Klappenschließkörper (8) aus biokompatiblem Material bestehen.

10. Verdrängerpumpe nach einem der vorangehenden Ansprüche, dadurch gekennzeichnet, daß Pumpengehäuse (1), Stutzen (9) und Klappengehäuse (7) aus thermoplastischem Polyurethan bestehen.

11. Verfahren zur Herstellung der Verdrängerpumpe nach einem der Ansprüche 1 bis 10, dadurch gekennzeichnet, daß Pumpengehäuse mit Stutzen und Klappengehäuse gemeinsam in einem Formvorgang hergestellt werden.

12. Verfahren nach Anspruch 11, dadurch gekennzeichnet, daß der Formvorgang ein Thermoformvorgang ist.

13. Verfahren nach Anspruch 11 oder 12, dadurch gekennzeichnet, daß es des weiteren den Schritt des Anformens der Klappenschließorgane durch Antauchen umfaßt.

14. Verfahren nach Anspruch 13, dadurch gekennzeichnet, daß gleichzeitig mit dem Anformen des Einlaßklappenschließorganes die Innenseite des Pumpengehäuses mit dem Material für die Klappenschließorgane beschichtet wird.

15. Verfahren nach einem der Ansprüche 11 bis 14, dadurch gekennzeichnet, es des weiteren den Schritt des Anformens der blutseitigen Membranlage durch Antauchen umfaßt.

16. Verfahren nach Anspruch 15, gekennzeichnet durch die in der nachfolgend wiedergegebenen Reihenfolge ablaufenden Schritte:

Formen des Pumpengehäuses mit integrierten Klappengehäusen;
Antauchen des Einlapklappenschließorganes mit gleichzeitiger Beschichtung der Innenseite des Pumpengehäuses;
Antauchen des Auslaßklappenschließorganes; und
Antauchen der blutseitigen Membranlage.

17. Verfahren nach einem der Ansprüche 11 bis 16, dadurch gekennzeichnet, daß das Pumpengehäuse in zwei Hälften hergestellt wird und daß die beiden Hälften nach der Anbringung der Klappenschließorgane und der Membran miteinander verspannt werden.

FIG. 1

EP 0 457 074 A1

FIG.2

Europäisches
Patentamt

**EUROPÄISCHER
RECHERCHENBERICHT**

Nummer der Anmeldung

**EP 91 10 6659**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,Y | GB-A-2 089 902 (NIPPON ZEON CO.)<br>* Seite 2, Zeile 43 - Zeile 59 * * Seite 3, Zeile 56 - Zeile 58 @ Seite 4, Zeile 46 - Zeile 50 @ Seite 5, Zeile 60 -Zeile 64 * * Seite 6, Zeile 50 - Seite 7, Zeile 10 * * Abbildungen 1,15A *<br>– – – | 1,2,8,10, 11,3,4 | A 61 M 1/10 |
| Y | US-A-3 656 873 (SCHIFF)<br>* Spalte 4, Zeile 41 - Zeile 73 * * Abbildungen 2,3 *<br>– – – | 3,4 | |
| X | DE-U-8 600 423 (MAGASI)<br>* Seite 11, Zeile 12 - Seite 12, Zeile 20 * * Seite 14, Zeile 1 - Zeile 21 @ Seite 15, Zeile 18 - Zeile 21 @ Abbildung 1 *<br>– – – | 1,5 | |
| X | EP-A-0 074 733 (UNIVERSITY OF UTAH)<br>* Seite 5, Zeile 32 - Seite 6, Zeile 22 * * Seite 7, Zeile 16 - Zeile 36 @ Seite 8, Zeile 19 - Zeile 30 @ Seite 9, Zeile 14 - Zeile 24 * * Seite 11, Zeile 27 - Seite 15, Zeile 17 * * Seite 16, Zeile 16 - Zeile 33 * * Abbildungen 1,5,6 *<br>– – – | 1,6,8,11, 12 | |
| X,A | US-A-4 863 461 (JARVIK)<br>* Spalte 11, Zeile 10 - Zeile 44 * * Spalte 12, Zeile 29 - Spalte 13, Zeile 11 * * Abbildungen 11-15 *<br>– – – | 1,6,7, 10-12,15, 5,17 | |
| A | US-A-4 573 883 (NOON ET AL.)<br>* Spalte 2, Zeile 31 - Spalte 3, Zeile 22 * * Abbildung 1 *<br>– – – – – | 5,17 | |

**RECHERCHIERTE SACHGEBIETE (Int. Cl.5)**

A 61 M

Der vorliegende Recherchenbericht wurde für alle Patentansprüche erstellt

| Recherchenort | Abschlußdatum der Recherche | Prüfer |
|---|---|---|
| Den Haag | 23 Juli 91 | SCHOENLEBEN J.E.F. |